(19) Europäisches Patentamt
European Patent Office
Office européen des brevets

(11) **EP 1 309 720 B1**

(12) **EUROPEAN PATENT SPECIFICATION**

(45) Date of publication and mention
of the grant of the patent:
**16.09.2009 Bulletin 2009/38**

(51) Int Cl.:
*C12Q 1/02* *(2006.01)*          *G01N 33/569* *(2006.01)*
*C12Q 1/00* *(2006.01)*          *G01N 33/00* *(2006.01)*

(21) Application number: **01984665.8**

(22) Date of filing: **11.07.2001**

(86) International application number:
**PCT/US2001/021820**

(87) International publication number:
**WO 2002/023188 (21.03.2002 Gazette 2002/12)**

(54) **APPARATUS AND METHOD FOR DETECTING AND CLASSIFYING CHEMICALS, PARTICLES, VIRUSES, AND BACTERIA IN FLUIDS WITH AN APTAMER**

VORRICHTUNG UND VERFAHREN ZUR BESTIMMUNG UND KLASSIFIZIERUNG VON CHEMIKALIEN, PARTIKELN, VIREN UND BAKTERIEN IN FLUIDEN MITTELS APTAMER

APPAREIL ET PROCEDE ASSURANT LA DETECTION ET LA CLASSIFICATION DE MATIERES CHIMIQUES, PARTICULES, VIRUS ET BACTERIES DANS DES FLUIDES AVEC UN APTAMER

(84) Designated Contracting States:
**AT BE CH CY DE DK ES FI FR GB GR IE IT LI LU MC NL PT SE TR**

(30) Priority: **11.07.2000 US 613942**

(43) Date of publication of application:
**14.05.2003 Bulletin 2003/20**

(73) Proprietor: **Chesapeake Techlabs, Inc.**
**Huntingon Valley, PA 19006 (US)**

(72) Inventors:
• **MANNIELLO, John**
  **Miami Beach, FL 33139 (US)**
• **LEIBHOLZ, Stephen**
  **Rydel, PA 19046 (US)**

(74) Representative: **Fischer, Franz et al**
**IPTO AG**
**International Patent and Trademark Office**
**Schwarztorstrasse 31**
**Postfach 5135**
**3001 Bern (CH)**

(56) References cited:
**WO-A-01/32911          WO-A-99/27351**
**US-A- 5 290 707          US-A- 5 526 685**
**US-A- 6 051 388          US-A- 6 087 114**
**US-A- 6 105 440**

• ROMANOVA N A ET AL: "BIOLUMINESCENCE OF FREE AND POLY(VINYL ALCOHOL) CRYOGEL-ENTRAPPED RECOMBINANT ESCHERICHIA COLI CELLS EXPRESSING FIREFLY LUCIFERASE" BIOCHEMISTRY, CONSULTANTS BUREAU, NEW YORK, NY, US, vol. 63, no. 5, May 1998 (1998-05), pages 579-583, XP000998782 ISSN: 0006-2979

## Description

FIELD OF THE INVENTION

The present invention is drawn to the detection of harmful agents, or agents in general, using a biosensor. More particularly, it is drawn to the use of bioreceptors for detecting and classifying chemicals, poisons, particles, vira, and bacteria in fluids. In a preferred embodiment, a bioreceptor selected from the group consisting of aptameric compounds is packaged in a collection device and treated to evoke an optical response to the presence of such agents, allowing for photocell detection and subsequent optional signal processing. These agents can also be collected in this manner for further analysis.

BACKGROUND INFORMATION

Chemical and biological weapons, sometimes referred to as the "poor man's nuclear weapons," pose a significant threat in the post-Cold War environment. The relative low cost and simplicity of their design and technology, in comparison to nuclear weapons, make them the weapons of mass destruction choice for a variety of rogue states and terrorist, non-state organizations. This threat has been made all the more tangible by the use of chemical agent in a Tokyo subway, and allegations over Iraq's development of chemical and biological weapons and its actual use of chemical weapons in combat operations.

According to the 1998 U.S Army Science und Technology Master Plan, "New fluorescent, acoustic, and optical biosensors are being designed for enhanced sensitivity and more flexible detection capability. Recent advances in the acceleration of the polymerase chain reaction (PCR) on a miniaturized scale now permit the exploitation of DNA probes for field detection of pathogens. A major thrust *of a Joint Warfighting Science and Technology Plan* (JWSTP) Defense Technology Objective (DTO), J.04 "Integrated Detection Advanced Technology Demonstration (ATD)," is the development of a rapid, automated field detection device based on the PCR. One key DTO element is the development of recombinant antibodies to serve as the recognition element of these new biosensors (FY98). Recombinant antibodies will ultimately be designed and quickly selected from genetic "super libraries" (FY99) to have specific detection capabilities, and novel starburst dendrimers are being studied for use on tailored reactive surfaces. Another major approach to point detection is mass spectrometry (MS), and miniature automated pyrolysis-based versions are being assessed for Integration into existing CBD platforms (FYO 1). Of critical importance for biosensor and MS approaches is bio-aerosol sampling, since characteristics (e.g., concentration of detectable units per unit volume of air) of biological aerosols differ dramatically from chemical vapors, with resulting effects of detection efficacy."

Of particular concern, which this invention addresses, are needs for (1) detecting, classifying and capturing agents heretofore undiscovered or unanalyzed, (2) annunciating these findings with a typical total processing time from exposure of about one second, (3) compactness and relatively low cost combined with long unattended life in the field, and (4) sensitivity to agents whose lethal or otherwise effective concentration in die environment will be extremely low compared to die comparable prior art.

US-A-6 051 388 discloses a biosensor comprising aptamers and its use.

US-A-5 526 685 proposes isokinetic sampling in the field of gas sampling.

BRIEF SUMMARY OF THE INVENTION

It is an object of the invention to use a bioreceptor for detecting and classifying chemicals, particles, vira, and bacteria in fluids.

It is yet another object of the invention to provide a system for directing harmful agents onto a sensor cell surface for use in detecting and classifying chemicals, particles, vira, and bacteria in fluids.

It is yet another object of the invention to provide diagnostic information and annunciation, either locally or remotely, within approximately 1 second or less of exposure to an agent in the free fluid, the time being short enough to become a function of the biochemical sensing apparatus alone.

It is another object of the invention to provide a aptameric compound packaged in a collection device and treated to evoke an optical response to the presence of harmful agents, allowing for photocell detection and/or collection of the harmful agents.

It is an object of the invention to use a bioreceptor which is an aptamer, for the detection of harmful agents.

It is another object of the invention to provide the functions of acquisition, filtering, sorting, selection, impingement on sensor cell surfaces and trapping of particles in a fluid, in accordance with desired mathematical functions of size, density, shape and surface characteristics.

It is another object of the invention to provide a combination of mechanical, aerodynamic, and kinetic methods to concentrate and/or sort particles of desired size and/or density from a fluid (gaseous, vapor or liquid) for detection by a sensor cell.

It is another object of the invention to provide a combination of mechanical, aerodynamic, and kinetic methods to impinge particles of desired size and/or density onto a bioreceptor surface for detection.

It is another object of the invention to provide a system for providing rapid annunciation of chemical, biological, and nuclear threats through interconnection of a plurality of biosensors via a central readout and decision system.

It is yet another object of the invention to provide a biosensor using aptameric compounds.

It is another object of this invention to provide broad annunciation capability combined with specificity, against agents that may not yet have been discovered by the emplacer of the biosensor

It is another object of this invention to achieve a sensing and processing time of about one second from exposure.

It is another object of this invention to combine compactness and low cost with long unattended life in the field.

It is another object of this invention to provide sensitivity to agents at very low concentrations in the environment.

It is a further object of this invention to provide for capturing the offending agents and furnishing a means for rapid preparation of antibodies to these agents.

BRIEF DESCRIPTION OF THE DRAWINGS

**Figure 1** discloses a basic schematic of the present invention.

**Figure 2** discloses a preferred intake port for the present invention.

**Figure 3** discloses a preferred embodiment of the present invention.

**Figures 4a-4c** disclose isokinetic separators used in the present invention.

**Figure 5** discloses an alternative embodiment of the present invention.

**Figure 6** discloses isokinetic flow model results for the present invention.

**Figure 7** discloses potential-based fluid flow mathematical simulation results for the aerodynamic sorting of the present invention.

**Figure 8** discloses a typical deployment scenario of the present invention.

DETAILED DESCRIPTION OF THE INVENTION

The present invention is to method and apparatus for directing harmful agents onto a bioreceptor surface for use in detecting and classifying chemicals, poisons, particles, vira, and bacteria in fluids, herein collectively referred to as *agents*. As used herein, the term *fluid* is intended to comprise, without limitation, any fluid including air and water, or fluids used as intermediates to convey agents to the device from solid objects or surfaces. The present invention incorporates a sensor for biological pathogens and poisons, chemical poisons, including neurotoxins and metabolic poisons, and drug or weapon precursor chemicals in the air or other fluids. The sensor is based on an aptamer treated with fluorescent or luminescent dye to evoke an optical response. The aptamer or sensor cell is selected and/or trained to respond to selected antagonists and ignore others.

In a preferred embodiment, the bioreceptor is an aptamer on a suitable organic substrate, protected from evaporation by a hydrophobic barrier or otherwise selective barrier or filter, porous to aerosols, particles and spores of a desired diameter and to gases dissolved in the in the atmosphere. A suitable hydrophobic barrier can be formed as a membrane or filter and can be applied at the intake, outlet, or covering the wells of the sensitive region. One suitable material for the hydrophobic barrier is polytetrafluoroethylene (PTFE) film with perforations of approximately 10$\mu$m in diameter.

The aptamers chosen and/or trained for this task should have certain properties discussed below. The term *trained* refers to any method for selecting or controlling the receptors or receptivity internal or external, of that aptamer. These properties:

- have minimal changes in sensor characteristics due to temperature, pressure or humidity changes
- respond to desired challenges and not respond to other challenges in selected patterns of response/non-response
- time-dependent processing of the readout data as detection and classification variables

- possess minimum sensitivity to temperature variations
- provide response to classes of challenges not previously catalogued and/or for which specific antibodies, DNA sequences or protein sequences may have or have not been determined
- provide means for generating antibodies to collected agents within the apparatus.

The biosensor of the present invention may incorporate Aristotelian and/or Non-Aristotelian logic, including multi-valued logic and statistical estimation to provide diagnostic information incorporating resistance to deliberately induced or accidental false alarms.

Each sensor may contain a replaceable plate or cartridge for holding the aptamer, with number of containers dependent on the deployed embodiment, such as in a rectangular or hexagonal configuration, with outlet holes for the air or other fluid interspersed among the aptamers or cells in the plate.

The biosensor container is generally in the form of a non-spherical optics subsystem and container, with reflective and/or refractive means to concentrate emitted photons to a small region external to the container. Each container terminates in a conventional photocell that collects the emitted photons resulting from activation of the aptamer either via luminescent response, such as, but not limited to, infused luciferase or aequorin, or via fluorescent response to infrared, ultraviolet or visual light stimulated emission. The collected photons are then converted to electrons by conventional photocell means and processed in a known manner to elicit space-time characteristics of the response. The results, incorporating both space-time characteristics of pattern response to activation of bioreceptors in individual containers, and the pattern of containers activated, are processed via appropriate Aristotelian and non-Aristotelian (probabilistic) logic to yield diagnostic signals.

Preferably, the challenging substance is also collected in the medium itself for further analysis. Additionally, the invention includes but is not limited to the use of shutters or trapdoors to control which bioreceptor containers are available to activation at a given time.

The invention includes the feasibility of rapid generation of antibodies using the device and captured samples, after exposure.

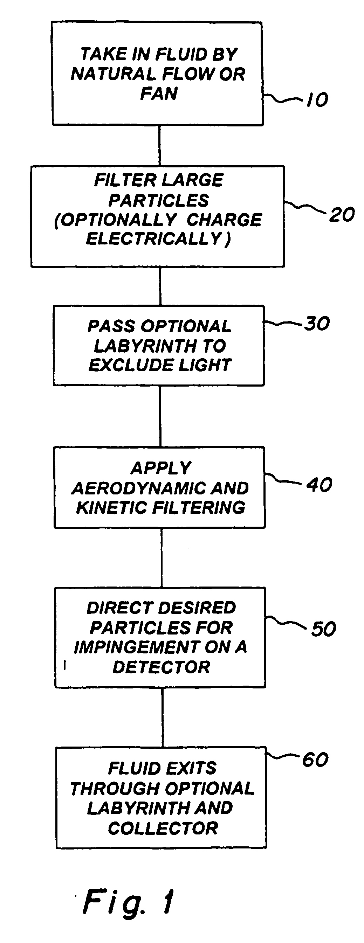

In its most basic form, as shown in **figure 1,** the present invention provides a means to concentrate and impinge particles, spores, vapors, and gasses from air or another fluid medium onto the bioreceptor. The fluid is taken in either through natural stream flows or via a small fan as shown at **10,** and passed through a filter designed to remove large particles from the fluid as shown at **20,** which may also incorporate the hydrophobic barrier discussed above. The resulting flow is then introduced into a chamber after optionally being electromagnetically charged and directed.

The impingement includes, without limitation, presenting pathogenic organisms, whether or not incorporated in aerosols, and/or toxins and poisons (including "poison gas" agents) suspended in the fluid as aerosols, vapors, gases, solutions, particles or by other means, onto sensitive surfaces of the bioreceptor intended to process the material, as shown at **50.**

The chamber consists of a series of collectors at one end, interspersed with one or more exit orifices. An optional sinusoidal or otherwise varying electrical field designed to optimally control the motion of the particles within the chamber sets up electrodynamic forces that cause the collectors, which are part of the electrodynamic apparatus, to attract the agents being collected and/or tested. This mechanism is discussed further below. The motion of the particles is generally in the form of a nonlinear vector motion and/or, but not limited to, a wave of increasing amplitude, terminating in impingement on the bioreceptor surface.

The remaining components of the fluid exit the chamber, and optionally pass through a finer filter to provide additional collection means.

The sensitive structure consists of a plate, volumetric receiver or array or other array of one or more sensitive bioreceptors of the present invention, each of which provides one or more components of the analysis that the entire system is assigned to accomplish.

The fluid passage optionally contains a series of designed bends or labyrinth, as shown at **30** whose purpose is to block light from the sensitive materials, as well as providing a portion of the aerodynamic filtering. This is in addition to the conventional mechanical filtering cited above.

The aerodynamic and kinetic filtering of **40** consists of one or more of three components. First, the bends in the passage are designed to employ a balance of centrifugal and drag forces to select out particles whose ratio of radius-squared to total mass (i.e. density times radius-cubed times four-thirds Pi) meets desired characteristics, mediated by the deliberately variable airflow in the bent section. Other mathematical functions serving as criteria for forting and selection include Bernoulli forces, and the effects of Reynolds and Froude Numbers as examples.

A structure bearing an aerodynamic functional resemblance to the Turbinate bone of vertebrate animals (found elsewhere in Nature as well) is optionally utilized to assure proper randomization of the components, as well as to cause early non-sensitive impingement of particles of undesired effective (i.e. aerodynamic) diameters.

In the preferred embodiment the optimum particle size for impingement is matched to the alveolar capture function, described below.

Each sensitive detector is optionally covered with a small trapdoor which when closed by electrical or mechanical means seals an associated detector. When open, the trapdoor optionally erects to a critical angle obtuse to the fluid stream, providing further optional selection of components of the stream using laminar or turbulent vortices to further provide discrimination for particle size and density.

The optional electromagnetic component of the device consists of a varying electrostatic field with incidental magnetic field optimized to accelerate particles of the appropriate size (surface area) and density onto the sensitive surface. The field either utilizes the induced dipole component of the particles being sorted or selected, or makes use of an electrostatic monopole on the each of the particles, using traditional methods for inducing the electrostatic monopole.

The fluid exits from the chamber (i.e., the chamber containing the plate or array and associated components) through holes in the plate or array interspersed among the detectors. The fluid then optionally passes through another light-restricting labyrinth to the exit and optional collection filter as shown at **60.**

In a preferred embodiment of the present invention, for combining with a sensor, the system has the responsibility of :

(1) extracting desired aerosol, vapor, gases and solid particles from the air,
(2) eliminating particles of inappropriate size (e.g., dust on one end and smoke on the other),
(3) sorting these particles while preserving viability of carried organisms, and
(4) concentrating and impinging the desired particles on the sensitive surface of the sensor.

The system can be broken down into four subsystems, not precisely corresponding to the above functions. It is optimized for acquisition of particles (aerosol or solid) forming particular mammalian threats, i.e. nominally 1-5$\mu$m in mean diameter.

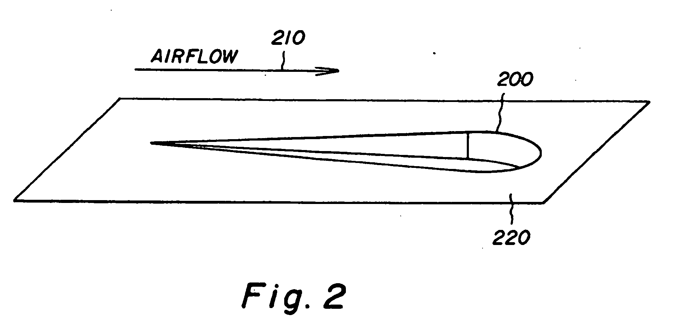

The first subsystem is concerned with particle acquisition and large-particle sorting. The challenge is that of capturing valid air samples despite wind direction and velocity. For this task the present invention preferably uses an omnidirectional assembly of the NACA flush intake design used in low-speed aircraft, which has the advantage of providing a positive Bemoulli-derived pressure to assure that sampling takes place. This has the further advantage of minimizing rain and small-particle effects, which could have an undesired effect on a sensor package. One such flush intake is diagramed in **figure 2** in which fluid such as airflow **210** is taken into NACA intake **200** in plate **220.** One such duct is of course partially unidirectional.

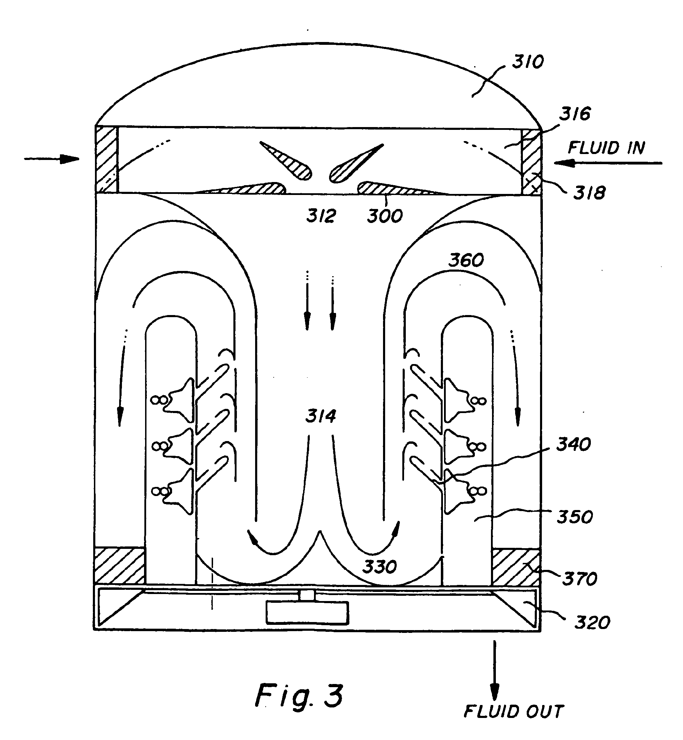

To act omnidirectionally, the system can use four or more such intakes **300** disposed around a circle, and covered by a hat or cover **310,** as shown in **figure 3.** These intakes **300** have a common (large-diameter) central opening **312,** with a smooth transition to a common intake **314.**

The main motive power for sampling is provided by a small DC-operated axial fan 320 moving approximately 15 L/m of air at very low pressure (pressure to be determined experimentally). This fan may be placed at the entrance or exit of the system.

The next subsystem concerns mechanical filtering. The entranceway **316** to these intakes 300 are covered by a mechanical dust filter **318** of high porosity. This filter **318** can be prepared using laser forming techniques and a hydrophobic base, available from Laserfare Inc. of Newport, RI. The design of the filter should be made to exclude particles of minimum diameter >10mm with minimum back-pressure.

The mechanical filtering components generally act on the mechanical minimum diameter of a particle. In the region of interest here, the Reynolds Number Re<1 and the orientation of an elongated particle is random. However, the primary filtering method to eliminate, for example, dust and smoke, must therefore act on the aerodynamic (actually Stokes) diameter of the particle, and therefore mechanical filtering is insufficient.

Note that from elementary geometric considerations derived from close-packing theory, the maximum theoretical porosity of such a filter can be shown to approximate $\dfrac{\pi}{2\sqrt{3}}$. Practical porosities of the order of 80% are therefore attainable, provided that a nonrandom filter-building process is utilized. A randomly generated filter (such as might be prepared chemically) has much lower porosity. The optimal design is that of the inversion of a hexagonal lattice in the X and Y dimensions (normal to the airflow) and a slightly bent tube in the Z direction. At the time of manufacturing, the laser-cut filters can be used to prepare molds for casting multiple copies, to reduce costs.

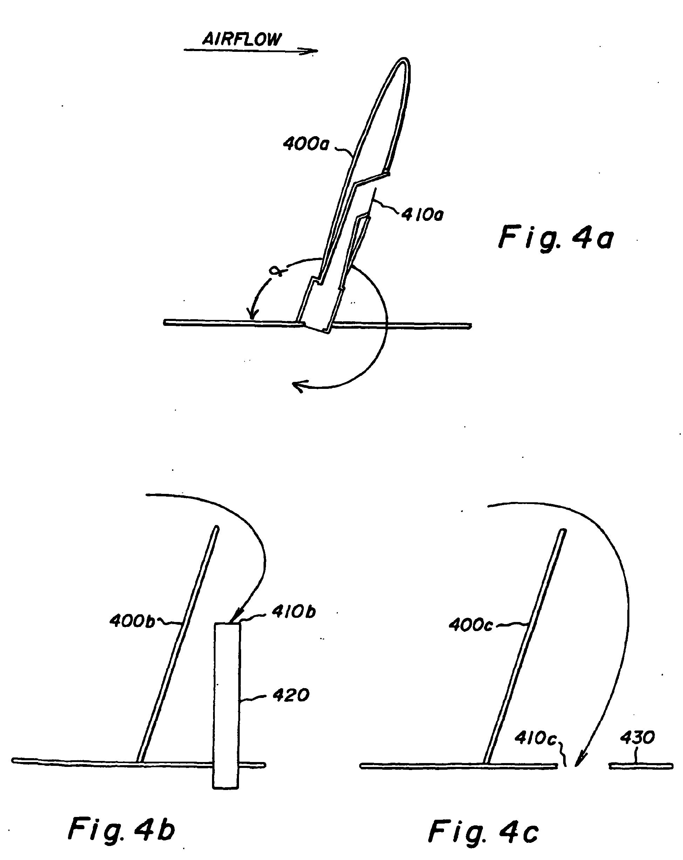

Because mechanical filtering alone is insufficient for desired system, the next subsystem concerns aerodynamic isokinetic filtering. Selection of particles having the desired aerodynamic diameters (as opposed to minimum diameters) is aerodynamic in nature. **Figures 4a-4c** illustrate the general dam mechanism as element **400a, 400b,** and **400c.** In **figure 3,** the fluid passes labyrinth **330** to pass over dams **340** for isokinetic filtering to collect particles of interest for impingement on the sensors of sensor array packages **350,** which are preferably replaceable. Remaining fluid passes exit labyrinth **360** to exit/collection filter **370** and fan **320.**

The design of the isokinetic filter is somewhat counterintuitive, involving dams **400a, 400b,** or **400c** at angles of attack (α) in the deep-stall region, i.e., greater than 90 ° in the preferred embodiment, but is borne out by research, analysis and computer simulation. In the regime of interest, aerosol particles having approximately the same density can be sorted by mean aerodynamic diameter quite efficiently. For the regions of interest, the diagram of **figures 4a-4c** are illustrative, and preliminary calculations have established the notional angle of attack of the aerodynamic filter.

In **figure 4a,** orifice **410a** acquires particles of interest for impingement onto a surface. It is integrated into the dam **400a** and is located on a backside (i.e., downstream side) thereof at a location appropriate for the desired isokinetic sorting. **Figures 4b** and **4c** disclose similar embodiments. In **figure 4b,** a collection tube **420** is used to position an orifice **410b** and in **figure 4c,** orifice **410c** is positioned on the base **430** adjacent to the dam **400c.**

The common aspect of all of these embodiments is that the orifice is at the downstream side of the dam or otherwise shielded from direct flow by the dam. Locating the orifice on the dam, on a separate collection tube, or on the base adjacent to the dam, but on the downstream side thereof, facilitates the isokinetic sorting. The ratio of stream velocity to orifice velocity, the angle of the dam, and the position and size of the orifice regulate the distribution of particles that enter the orifice. This is demonstrated in the simulation.

Although orifices **410a** and **410c** will generally be fixed, orifice **410b** can optionally be easily repositioned by making collection tube **420** movable. For example, although there is no functional difference between an orifice **410a** in the dam **400a** and an orifice **410b** on a collection tube **420** positioned to be shielded by the dam **400b** from the prevailing flow vector, the angle of collecting tube **420** with respect to the base (i.e., X-axis) is also a solution variable. Sorting can be varied based on repositioning of the collection tube **420**. Dam **400c** can also have utility as a trap door to cover a sensor.

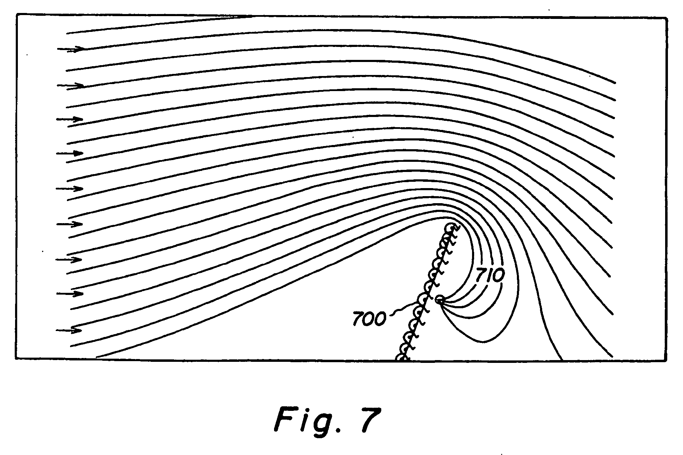

Basically, as shown in **figure 7,** the dam **700** sets up a local vortex **710** which sorts the particles isokinetically, by density and aerodynamic diameter. The underlying theory is also discussed below, with preliminary analysis of the phenomenon, both extrapolated from experimental data and supported by a first-order simulation.

The final subsystem concerns impaction. After some analysis, the present inventors have concluded that the conventional aerodynamic impaction methodologies exhibited in commercial off-the-shelf viable-particle impactors are satisfactory and probably optimal for the present application. An existing impaction chamber design, such as those available from Anderson Instruments, 500 Technology Court, Smyrna, GA 30082, USA, is therefore usable for the present invention.

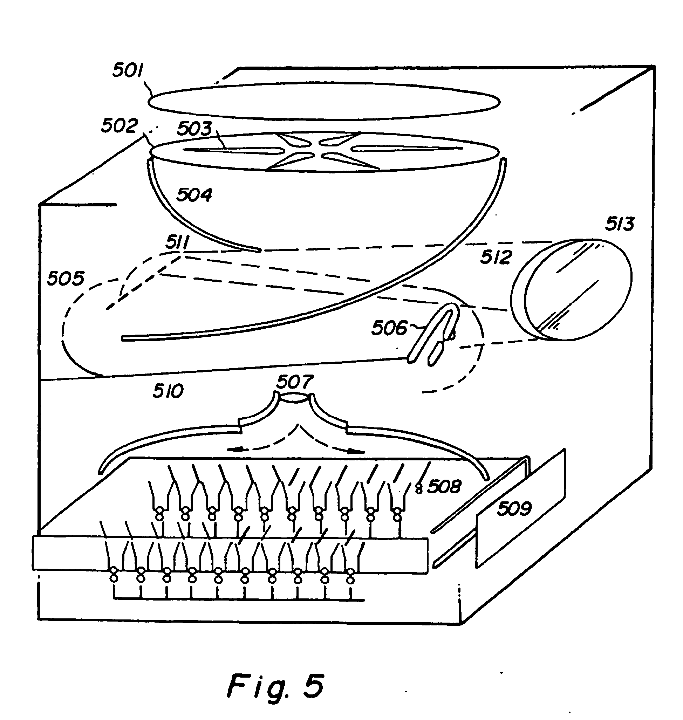

**Figure 5** illustrates an alternate embodiment of the invention in which such an impaction chamber **507** is employed. In this embodiment, large particles and rain are excluded by input rain and sun hat **501** and large particle filter **502**. Fluid then passes through NACA fluid dynamic intake orifices **503** to a collector and second filter **504** to an intake labyrinth and venturi **505** that directs the fluid over fluid dynamic dam **506** which acts as an inertial filter to obtain particles of interest at an orifice at the rear. Particles of interest are directed by inertial impactor **507** (which can optionally be replaced with the electrodynamic impaction discussed above) over the open trap doors of sensors **508** which are optionally replaceable via drawer **509**. The remaining fluid is collected in chamber 510, passed through exit labyrinth **511** to fan **512** to exit collector and filter **513**.

The apparent particle density of an aerosol is generally much smaller than the actual density, with coefficients of sphericity (ratio of surface area of an equivalent sphere to actual surface area $(A/A_0)$). This means that for organic aerosols the apparent density is uniformly less than unity, and in the range where Stokes' Law does not apply experimentally very well. For the region of interest it amounts to a correction to Stokes' Law, and the inventors have chosen to use experimental data as guidance. Below, it is noted that preliminary simulation results using the assumptions underlying Stokes' Law are quantitatively consistent with the extrapolated experimental data. Robinson (see *Comm. Pure Applied Math*, 9,69) demonstrated that in these cases gravitational and inertial collection efficiencies are additive.

From the point of view of alveolar deposition (which is most critical from an infection viewpoint, and therefore defines both threat and measurement),the most recent data from Lippmann (see Lippmann, M. "Regional Deposition of Particles in the Human Respiratory Tract", in Handbook of Physiology, American Physiological Society, Bethesda, MD 1977) suggests that for both oral and nasal alveolar deposition the optimum aerodynamic diameter is of the order of 1-5 μm, with tails of about .7-7 μm at the 15% efficiency level.

Without belaboring the derivation, from Stokes' Law and the assumptions of low Reynolds Number it can be shown that gravitational settling is fairly independent of wind velocity (including turbulence) and is given by

$$Re^2 \varphi = \frac{8 * [(4/3)\pi r^3]\gamma_a \gamma g}{\pi \eta^2}$$

where Re is the Reynolds Number, which by definition= $2r\gamma_a V / \eta$ yielding the velocity V, and where
γ = density of the particle
γα = density of the air
g = gravitational acceleration

η = viscosity of the air

There are better approximations based on empirical data, but for this analysis and design purposes they can be ignored unless it is required to accurately measure particle density and mean radius.

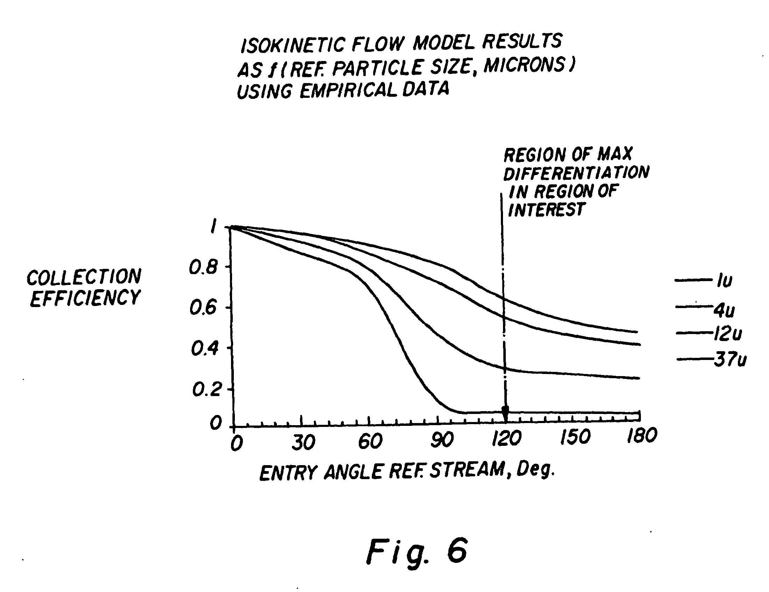

Data from Watson (see *Amer. Ind Hyg. Ass. Quarterly*, 3,29) is shown in **figure 6,** which has been nondimensionlized (appropriate in the region of interest) has been extrapolated by the inventors to cover the expected particle size range ($\rho_{eff} \approx$ 1), indicating how the sorting mechanism will select out particles of the appropriate size. These data are fortunately nondimensional, and confirm the present design and analysis. Most importantly, smoke particles, having effective diameters well under .5 $\mu$m will be rejected.

For the aerodynamic sorting, the entire air-handling problem is properly modeled by the Navier-Stokes formulation.

$$\left[\frac{\partial u_i}{\partial t}\right] \div \left[\frac{\partial u_i}{\partial x_j}\right] \bullet [u] = -\frac{1}{\rho}\left[\frac{\partial p}{\partial x_j}\right] + \begin{bmatrix} 0 \\ 0 \\ -g \end{bmatrix} + \frac{\mu}{\rho}\left(\sum_i \left[\frac{\partial^2 u_i}{\partial x_i^2}\right] + \frac{1}{3}\left[\frac{\partial^2 u_l}{\partial x_l \partial x_j}\right]\right)$$

supplemented by the Equation of Continuity (incompressible flow in this case) and the thermodynamic relationship (generally adiabatic flow with $\gamma \approx$ 1.5, but also negligible in this instance).

Using a potential-based fluid flow simulator, available from the U. Mich. Department of Aerospace Engineering, a simple model of an appropriate obstruction to uniform flow was modeled at an appropriate angle. The results are illustrated in **figure 7.** Assumptions included incompressibility, and irrotational laminar flow. These assumptions are appropriate to the scenario including the prevailing Reynolds numbers (<<1). A number of runs were made, varying appropriate parameters (i.e., ratio of source to sink rates and angle of incidence), to demonstrate the aerodynamic filtering mechanism.

The airfoil angle of incidence in the pictured simulation was 210°. The nondimensional input flow rate of the pictured simulation run was 1 sec$^{-1}$, and the sink rate was located at a notional fluid sink of 2 sec$^{-1}$ at the ventral center of an arbitrarily thin barrier. Other ratios of source to sink rate (1:1 to 1:3) provided similar results. The streamlines are shown. The equipotential lines are of course orthogonal to the streamlines, and reveal no surprises.

Our analysis has shown that the optimal incidence angle may vary from the incidence angle shown, including angles in the laminar, turbulent and stall regions, depending on the selection criteria, densities, velocities, and Reynolds numbers. Furthermore the collection orifice, while not directly in a streamline, may be partially so, located on the barrier, or on the substrate of the barrier, again depending on the conditions of design and operation. The class of inlets used for optimum pressure recovery, referred to as "NACA" or NACA-type" are generally unsuitable to this solution.

From these results, it is fairly obvious under the conditions in which Stokes' Law is valid (these being the case) that, to the first order, a particle of density ρ with a mean radius of embedded in a much lighter fluid ($\rho_f << \rho_p$) having a curved path with radius $r_a$ and a flow velocity of $v_a$ will have a terminal velocity component $v_p$ normal to the fluid streamlines (neglecting gravity, which can be vectorially added later) of:

$$v_p = \frac{4\rho r^2 v_a^2}{18\mu r_a} = K\frac{\rho r^2 v_a^2}{r_a}$$

which provides square-law sorting by particle size. Varying the incidence angle of course changes the flow and the local curvature of the streamlines, and the present invention anticipates an incidence angle of 180<α<270 (see simulation graphic) for particle sorting, and, from the simulation, for the stated conditions, α≅210 degrees seems optimal to provide the greatest dispersion of particle paths. Thus there is an approximate agreement between the extrapolated empirical data and the simulation.

The exact dam and entry angles can vary from those disclosed herein. (Note that 120° in the empirical diagram corresponds geometrically to an incidence angle of 210°.) This computational experiment merely serves (1) to demonstrate qualitatively the measured filtering effects and (2) provide a basis for detailed analytical simulation in the course of the project to optimize the aerodynamic sorting phase of the preferred embodiment of the present invention.

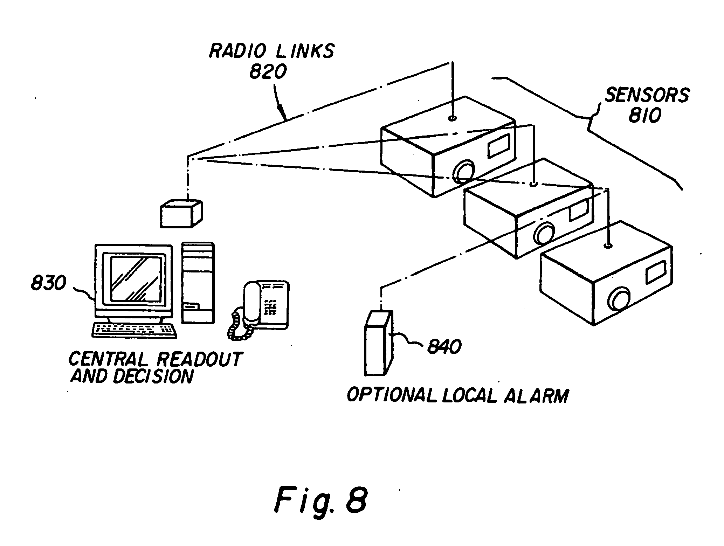

In use, the sensors of the present invention can be configured to be hand emplaceable, as shown in **figure 8,** or wearable (not shown). In a preferred embodiment, sensor units **810** are deployed and are linked, such as by radio links **820,** to a central readout and decision center **830.** If the sensor units **810** are deployed with military units or near populations, a local alarm **840** can be used to warn personnel.

An alternative deployment involves groups of individuals each carrying a wearable sensor sensitized for and optimized for a smaller class of agents. The aggregate wearable sensors in this group communicate locally among each other via low-powered radio signals, forming a self-organizing redundant network, to achieve a combination of selectivity and breadth of response. The aggregate results are then optionally communicated to the central readout and decision center.

The central readout and decision center **830** can (1) provide detection and classification of the agent, depending on the strength and signal time-characterizations reported by each well or container, and the pattern of responses among all the containers within the apparatus, (2) minimize both false negatives and false positives in the decision, (3) optionally deal with uncertainty or unanticipated patterns of response-including spoofing and obscuration-according to probabilistic rules, (4) compose an annunciation and diagnostic report message, and (5) transmit that message or annunciation by conventional means.

The underlying software algorithms were separately developed by one of the present inventors (Leibholz) and the software is protected as a copyright.

Aptamer-based bioreceptors:

The general process of the present invention for using aptamer-based bioreceptors is analogous to that of the use of genetic algorithms to construct the desired sequences. The affinity of a putative aptamer to a target is determined by geometric as well as chemical considerations, being a mixture of folding properties, multipole electrostatic and van der Waals forces, generally both covalent and noncovalent bonds. The photonic conversion reporter process is a result of polarization changes rather than $Ca^{++}$ channel changes in the sensor biomolecules.

A sensor aptamer of the present invention can be produced and deployed, for example, by the following method:

A. Develop targets of interest (e.g., JCS list agents, bacteria, pollens, viruses, prions, metabolic toxins, other pathogens, narcotic precursors and by-products, et cetera) by using infective and replication processes known in the literature;

B. Collect T-cell and B-cell populations where appropriate;

C. Prepare a large number of pseudorandom sequences of nucleic acids;

D. Mix them with the targets in solution phase;

E. Discard the unbound or weakly bound molecules, e.g., via electrophoretic or chromatographic methods;

F. Continue process of collecting adhering candidates, further modification through randomization and resorting proceeds until a desired degree of affinity is obtained;

G. Attach the photonic reporter molecules;

H. Attach the combined molecules to the substrate, typically a decelularized biomembrane; and

I. Test and repeat.

This invention therefore provides for sensing biological, chemical or radiation challenges to humans, animals, and plants even when that challenge has not previously been catalogued, isolated and/or analyzed. It also can be used to reliably detect biological, chemical or radiation challenges to humans, animals and plants despite the presence of natural or deliberate mutations, and/or conformers, enantiomers and/or chimeric agents.

A technology that is useful with the present invention is the control of nuclear envelope breakdown (NEB) in the sensor cells, both to speed up cell production prior to deployment and to enhance cell use after deployment.

From earlier published documents, it is known that cells produce and probably require a discrete calcium signal prior to nuclear envelope breakdown. This calcium signal is expressed from intracellular endomembranes acting as the sink and source for the calcium signal.

From earlier published documents, the calcium-uptake activity of ATP-dependent calcium pump enzyme of the endoplasmic reticulum (ER) is required for cell division.. The calcium signal is expressed as a discrete space-time pattern.

From earlier published documents, for inhibition of cell division, disruption of the space-time pattern of the pre-nuclear-envelope-breakdown (pre-NEB) calcium signal is employed which results in cessation of nuclear envelope breakdown and thus cell division.

From earlier published documents, the enzymes essential to produce cell division are present as part of the peri-nuclear endomembranes. A selective, properly timed inhibition of any of the enzymes needed to produce this cell division, particularly leukotriene B4 (e.g., phospholipase A2, 5-lipoxygenase, leukotriene A4 hydrolase) results in blocking of NEB and subsequent cell divisions.

From earlier published documents, the production of leukotriene B4 is part of an integrated network of enzymes spanning the 5-lipoxygenase, glutathione reductase/glutathione S-transferase-leukotriene c4 synthase, pentose phosphate and glycolytic pathways, and likely involves feedback with purine, pyrimidine and amino acid biosynthesis. Thus, disruption of the space-time pattern of the pre-NEB calcium signal either by superimposing a random or other pattern, or disrupting biosynthesis of leukotriene B4 will result in blocking of nuclear envelope breakdown and cell division.

For initiation or enhancement of the probability of cell division, the space-time pattern of the pre-NEB calcium signal is mimicked or enhanced, using open-loop chemical, photonic or electrical means, or using closed-loop augmentation of the natural signals.

Such a disruption, properly applied in space-time, is can thus be used to stop cell division (e.g., to terminate a cancer, et cetera). This disruption may take the form of continuous (DC) or random signals, as well as periodic signals of appropriate frequency, waveform and amplitude.

Similarly, expression of such a space-time patterned signal as normally occurs in normal cells, maybe used to restore the cell division process in cells that have stopped cell division (e.g., aplastic anemia, et cetera) or in which controlled cell division needs to be restored (e.g., regeneration of burned tissues, et cetera). This signal may be generated in open-loop or closed-loop fashion, either creating the desired response or enhancing it.

**Claims**

1. A method of biosensing an agent by collecting, sorting, concentrating and impinging particles on a surface of a bioreceptor, comprising:

   capturing a fluid sample by drawing it through an intake;
   mechanically and/or aerodynamically filtering out particles > 10$\mu$m;
   using a dam to set up a local vortex to isokinetically sort particles by density and aerodynamic diameter; and
   selecting particles within a selected size-range of said agent for impaction on the surface of the bioreceptor, wherein said bioreceptor is an aptamer.

2. The method of claim 1, further comprising providing a photocell, wherein said bioreceptor is treated with fluorescent or luminescent dye to evoke an optical response in
   the presence of said agent so as to be detected by said photocell.

3. The method of claim 1, wherein said selected size range is 0.7-7 $\mu$m.

4. The method of claim 1, wherein said dam has an angle of attack between 180 and 270 degrees with respect to said fluid.

5. An apparatus for biosensing an agent by collecting, sorting, concentrating and impinging particles on a surface of a bioreceptor, comprising:

   a fluid intake;
   a mechanical filter with an effective mesh size of 10 $\mu$m; and
   a fluid flow conduit having at least one dam means to set up a local vortex to isokinetically sort particles by density and aerodynamic diameter, wherein said dam means includes means for selecting particles within a selected size-range for impaction on a bioreceptor surface,

   wherein said bioreceptor surface contains an aptamer.

6. The apparatus of claim 5, further comprising a photocell, wherein said sensor biomolecules have been treated with fluorescent or luminescent dye so as to evoke an optical response in the presence of said agent to be detected by said photocell.

7. The apparatus of claim 5, wherein said mechanical filter includes a labyrinth element to exclude light.

8. The apparatus of claim 5, wherein said dam means has an angle of attack between 180 and 270 degrees with respect to said fluid.

9. The apparatus of claim 5, wherein said dam means includes an upstream dam element and said means for selecting particles is an orifice on a downstream side of said dam element, wherein said orifice is located at a position selected from the group comprising a downstream side of said dam element, an end of a collection tube located downstream of said dam element, and a base adjacent a downstream side of said dam element.

10. The apparatus of claim 5, further comprising an interconnection of a plurality of biosensing apparatuses via a central readout and decision system.


**Patentansprüche**

1. Verfahren zum Biosensing eines Agens durch Sammeln, Sortieren, Konzentrieren und Aufschlagen von Partikeln auf eine Oberfläche eines Biorezeptors, enthaltend:

Erfassen einer Flüssigkeitsprobe indem sie durch einen Einlass gesogen wird;
mechanisches und/oder aerodynamisches Filtern der Partikel > 10 $\mu$m;
Verwenden eines Dammes um einen lokalen Wirbel aufzubauen um isokinetisch Partikel durch Dichte und aerodynamischen Durchmesser zu sortieren; und
Auswahl von Partikeln innerhalb eines ausgewählten Grössenbereichs des genannten Agens für den Aufprall auf die Oberfläche des Biorezeptors,

wobei der Biorezeptor ein Aptamer ist.

2. Verfahren nach Anspruch 1, weiter enthaltend die Bereitstellung einer Photozelle, worin der genannte Biorezeptor mit fluoreszierendem oder lumineszierendem Farbstoff behandelt wird um eine optische Reaktion zu bewirken, in Gegenwart des genannten Agens, damit es durch die genannte Photozelle nachgewiesen werden kann.

3. Verfahren nach Anspruch 1, worin der genannte Grössenbereich 0,7 -7 $\mu$m ist.

4. Verfahren nach Anspruch 1, worin der genannte Damm einen Angriffswinkel von 180 bis 270 Grad bezüglich des genannten Fluids aufweist.

5. Apparat zum Biosensing eines Agens durch Sammeln, Sortieren, Konzentrieren und Aufschlagen von Partikeln auf eine Oberfläche eines Biorezeptors, enthaltend:

eine Flüssigkeitsaufnahme
ein mechanisches Filter mit einer effektiven Maschenweite von 10 $\mu$m; und
eine Fluidfliessleitung, die zumindest ein Damm-Mittel aufweist um einen lokalen Wirbel zu aufzubauen um Partikel isokinetisch nach Dichte und aerodynamischem Durchmesser zu sortieren, wobei das genannte Damm-Mittel ein Mittel zur Selektierung von Partikeln innerhalb eines ausgewählten Grössenbereichs enthält, für den Aufprall auf eine Biorezeptoroberfläche,

wobei die genannte Biorezeptoroberfläche ein Aptamer enthält.

6. Apparat nach Anspruch 5, enthalten weiter eine Photozelle, worin die genannten Sensor-Biomoleküle mit fluoreszierendem oder lumineszierendem Farbstoff behandelt worden sind um eine optische Reaktion, in Gegenwart des genannten Agens, das durch die genannte Photozelle nachgewiesen werden soll, zu bewirken.

7. Apparat nach Anspruch 5, worin das genannte mechanische Filter ein Labyrinth-Element enthält um das Licht auszuschliessen.

8. Apparat nach Anspruch 5, worin das genannte Damm-Mittel einen Angriffswinkel zwischen 180 und 270 Grad bezüglich des genannten Fluids hat.

9. Apparat gemäss Anspruch 5, worin das genannte Damm-Mittel ein Stromaufwärts-Dammelement enthält und das genannte Mittel zum Selektieren von Partikeln eine Öffnung auf der Stromabwärts-Seite des genannten Dammelements ist, wobei die genannte Öffnung sich in einer Position befindet, die ausgewählt ist aus der Gruppe enthaltend die Stromabwärtsseite des genannten Dammelements, ein Ende eines Sammelrohrs, das sich stromabwärts vom genannten Dammelement befindet, und eine Basis, die an die Stromabwärtsseite des genannten Dammelements anliegend ist.

10. Apparat nach Anspruch 5, enthaltend weiter eine Interkonnektion einer Mehrzahl von Biosensing-Apparaten über ein zentrales Ablese- und Entscheidungssystem.

## Revendications

1. Procédé pour la biodétection d'un agent en collectionnant, en triant, en concentrant et en percutant contre une surface d'un biorécepteur, ce procédé comprenant:

   capturer un échantillon de fluide en le tirant par une entrée ;
   extraire des particules > 10 μm par filtration mécanique et/ou aérodynamique ;
   utiliser une digue pour former un tourbillon local pour trier des particules isocinétiquement en fonction de la densité et du diamètre aérodynamique ; et
   sélectionner des particules dans une étendue de taille sélectionnée de l'agent mentionné pour l'impact sur le surface du biorécepteur, où le biorécepteur mentionné est un aptamère.

2. Procédé selon la revendication 1, comprenant ultérieurement la mise à disposition d'une photocellule, dans laquelle le biorécepteur est traité avec un colorant fluorescent ou luminescent pour évoquer une réponse optique.

3. Procédé selon la revendication 1, dans lequel l'étendue de taille sélectionnée est 0,7 à 7 μm.

4. Procédé selon la revendication 1, dans lequel la digue mentionnée a un angle d'attaque entre 180 et 270 degrés.

5. Appareil pour la biodétection d'un agent en collectionnant, en triant, en concentrant et en percutant contre une surface d'un biorécepteur, cet appareil comprenant :

   une entrée de fluide
   un filtre mécanique avec une taille de maille effective de 10 μm ; et
   une conduite à flux de fluide comprenant au moins un moyen de digue pour former un tourbillon local pour trier des particules isocinétiquement en fonction de la densité et du diamètre aérodynamique, dans laquelle la digue mentionnée comprend des moyens pour sélectionner des particules dans une étendue de taille sélectionnée pour l'impact sur la surface du biorécepteur,

   où la surface du biorécepteur mentionnée comprend un aptamère.

6. Appareil selon la revendication 5, comprenant ultérieurement une photocellule, dans laquelle les biomolécules de senseur mentionnés ont été traités avec un colorant fluorescent ou luminescent pour évoquer une réponse optique en présence de l'agent mentionné à détecter par la photocellule mentionné.

7. Appareil selon la revendication 5, dans lequel le filtre mécanique mentionné comprend un élément de labyrinthe pour exclure la lumière.

8. Appareil selon la revendication 5, dans lequel le moyen de digue mentionné a un angle d'attaque entre 180 et 270 degrés relative au fluide mentionné.

9. Appareil selon la revendication 5, dans lequel le moyen de digue mentionné comprend un élément de digue en remontant le courant et le moyen pour sélectionner des particules est une ouverture à un côté en descendant le courant de l'élément de digue mentionné, où l'ouverture mentionnée est située dans une position sélectionnée du group comprenant un côté en descendant le courant de l'élément de digue mentionné, un bout d'un tube de collection situé en descendant le courant de l'élément de digue mentionné et une base située à côté de l'élément de digue mentionné.

10. Appareil selon la revendication 5, comprenant ultérieurement une interconnexion d'une pluralité des appareils à biodétection par un système de lecture et de décision.

TAKE IN FLUID BY
NATURAL FLOW OR
FAN                           ～10

FILTER LARGE
PARTICLES
(OPTIONALLY CHARGE      ～20
ELECTRICALLY)

PASS OPTIONAL      ～30
LABYRINTH TO
EXCLUDE LIGHT

APPLY
AERODYNAMIC AND      ～40
KINETIC FILTERING

DIRECT DESIRED
PARTICLES FOR
IMPINGEMENT ON A      ～50
DETECTOR

FLUID EXITS      ～60
THROUGH OPTIONAL
LABYRINTH AND
COLLECTOR

*Fig. 1*

**AIRFLOW** *210* → 

*200*

*220*

# Fig. 2

Fig. 3

AIRFLOW

400a

410a

*Fig. 4a*

α

400b

410b

420

*Fig. 4b*

400c

410c

430

*Fig. 4c*

Fig. 5

EP 1 309 720 B1

ISOKINETIC FLOW MODEL RESULTS
AS f ( REF. PARTICLE SIZE, MICRONS )
USING EMPIRICAL DATA

Fig. 6

Fig. 7

Fig. 8

RADIO LINKS
820

SENSORS
810

830

CENTRAL READOUT
AND DECISION

840

OPTIONAL LOCAL ALARM

EP 1 309 720 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6051388 A
- US 5526685 A

**Non-patent literature cited in the description**

- Regional Deposition of Particles in the Human Respiratory Tract. **Lippmann, M.** Handbook of Physiology. American Physiological Society, 1977